# EUROPEAN PATENT APPLICATION

(11) **EP 2 327 397 A2**
(43) Date of publication of application: **01.06.2011**
(21) Application number: 09810220.5
(22) Date of filing: 28.08.2009
(51) Int. Cl.: A61K 9/16

(54) **METHOD FOR MANUFACTURING SUSTAINED RELEASE MICROSPHERE BY SOLVENT FLOW EVAPORATION METHOD**

(30) Priority: 29.08.2008 KR 20080085368
(71) Applicant: Dongkook Pharmaceutical Co., Ltd., Seoul 135-502 (KR)
(72) Inventor: LIM, Nak Hyun, Suwon Gyeonggi-do 443-717 (KR); KIM, Sung Geun, Suwon Gyeonggi-do 443-717 (KR); KIM, Se Yeon, Suwon Gyeonggi-do 443-725 (KR); JUNG, Hyung Joon, Suwon Gyeonggi-do 443-717 (KR); CHA, Kyung Hoi, Yongin Gyeonggi-do 446-754 (KR)
(74) Representative: Kador, Ulrich
(86) International application number: PCT/KR2009/004820
(87) International publication number: WO 2010/024615

(57) **Abstract**

The present invention relates to a method for preparing a sustained-release microsphere which can control the long-term release of a drug. More particularly, as the preparation of a microsphere in which a drug is loaded in a carrier comprising a biodegradable polymer, the present invention relates to a method for preparing a sustained-release microsphere wherein a solvent intra-exchange evaporation method by means of co-solvent is used for suppressing the initial burst release of physiologically active substance, to release the physiologically active substance in the body continuously and uniformly.

## Description

### TECHNICAL FIELD

The present invention relates to a method for preparing a sustained-release microsphere which can control the long-term release of a drug. More particularly, as the preparation of a microsphere in which a drug is loaded in a carrier comprising a biodegradable polymer, the present invention relates to a method for preparing a sustained-release microsphere wherein a solvent intra-exchange evaporation method by means of co-solvent is used for suppressing the initial burst release of physiologically active substance, to release the physiologically active substance in the body continuously and uniformly.

### BACKGROUND ART

Up to now, a coacervation method, a melt extrusion, a spray drying method and a solvent evaporation method have been known as general methods for preparing a sustained-release injectable formulation. Among such methods, the solvent evaporation method, which is classified as a double-emulsion solvent evaporation method (W/O/W, water/oil/water) and single-emulsion solvent evaporation method (O/W, oil/water), has been commonly used.

To improve such a sustained-release injectable formulation, there have been many attempts such as increasing of drug loading efficiency, the simplification of preparation process and the lowering of initial burst release. Korean Patent Application No. 2003-0023130 discloses a method for producing a sustained-release nanoparticle comprising the steps of dissolving a biodegradable polymer and a drug in a solvent, spraying a solution by using a spray drying method with a constant flow, and drying under reduced pressure. However, the above invention has drawbacks, such as the cost of installing an expensive spray dryer, aseptic treatment of equipment, as well as initial burst release of physiologically active substance.

Korean Patent Application No. 2004-7009999 discloses a method of producing a microsphere wherein an osmotic pressure-regulating agent is added to increase drug loading to the microsphere and improve dispersibility of the microsphere. However, the above invention still has a disadvantage of initial burst release due to the increase of drug amount distributed on the surface of the microsphere caused by over-loading of the drug.

Korean Patent No. 0409413 discloses a method of producing a sustained-release microcapsule wherein the microcapsule is prepared by an in-water drying method, and then the obtained microcapsule is thermally dried at a temperature not lower than the glass transition temperature of a biodegradable polymer to suppress the initial release of the bioactive substance in excess and to minimize retention of organic solvent. However, the above invention has a drawback of heat denaturation of the bioactive substance.

US Patent No. 5,366,734 discloses a method of preparing a formulation for continuously administering a pharmaceutically active peptide wherein the mixture of the drug and biodegradable polymer is solidified in implant form and then the implant is coated. However, such an implant is too large, and thus the needle size for subcutaneous injection would have to be enlarged. As a result, a patient may feel fear and local anesthesia would be needed due to pain when it is injected.

### TECHNICAL PROBLEM

The present invention is conceived to address the above problems. The technical problem to be solved by the present invention is to provide a method for preparing a sustained-release microsphere which has no problems in the conventional techniques and efficiently suppresses the initial burst release of physiologically active substance.

### SOLUTION TO PROBLEM

To accomplish the above object, the present invention provides a method for preparing a sustained-release microsphere having an excellent suppression against the initial burst release of physiologically active substance which comprises adding co-solvent to an emulsion including a physiologically active substance and a biodegradable polymer, and adding the emulsion to an aqueous solution.

In addition, the present invention provides a sustained-release microsphere prepared according to the above preparation method.

The present invention is described in detail hereinafter.

The present invention provides a method for preparing a sustained-release microsphere comprising the steps of:
i) dissolving a physiologically active substance and a biodegradable polymer in an aqueous solvent and a non-aqueous solvent, respectively, and then additionally dissolving a surfactant in the aqueous solvent, the non-aqueous solvent, or both the aqueous solvent and the non-aqueous solvent;
ii) forming a emulsion by combining the aqueous solution and the non-aqueous solution of step (i);
iii) adding a co-solvent to the emulsion formed in step (ii)
iv) forming a microsphere by adding the emulsion of step (iii) to an aqueous solvent; and
v) removing an organic solvent.

In the present specification, the above method is referred to as a "solvent intra-exchange evaporation" method.

In the present invention, the physiologically active substance is not specifically limited and is preferably selected from a peptide drug such as luteinizing hormone-releasing hormone (LHRH) analogue or salt thereof. For example, among the LHRH analogues, an agonist includes goserelin, leuprolide, triptorelin, buserelin, nafarelin and the like, and an antagonist includes cetrorelix. In addition, other available peptide drugs include octreotide and the like. The agonist of LHRH analogues is a peptide molecule which suppresses the secretion of sex hormones-testosterone and estrogen- by suppressing the secretion of luteinizing hormone by means of acting on the pituitary gland when it is administered (the agonist initially promotes the secretion, but it suppresses the secretion when it is continuously released). As a result, the agonist of LHRH analogues has treatment effects on prostate cancer, breast cancer, endometriosis, etc., which are hormone-sensitive.

In the present invention, the physiologically active substance may be comprised as the amount of 1.0 to 30% by weight, preferably 2.0 to 20% by weight, and more preferably 3.0 to 15% by weight based on total weight of the microsphere. If the amount of the physiologically active substance is less than 1.0% by weight, the amount of microsphere to be administered is too large to inject or may be problematic at the time of injection. If the amount of the physiologically active substance exceeds 30% by weight, it is difficult to suppress the initial burst release.

In the present invention, the aqueous solvent used for dissolving the physiologically active substance in step (i) may be water for injection.

In the present invention, a surfactant may be added to the aqueous solvent and/or the non-aqueous solvent of the above step (i). Available surfactant includes, but is not limited to, polysorbate, Span 80, poloxamer, polyethylene glycol, tocopherol and the like. In the present invention, the surfactant may be added in the amount of 0.01 to 5.0 parts by weight based on 100 parts by weight of total weight of the aqueous solution and the non-aqueous solution. If the amount of surfactant is less than 0.01 parts by weight, there may be a problem in forming an emulsion due to the decreasing effects of the surfactant. If the amount of surfactant exceeds 5.0 parts by weight, the large amount of the surfactant may cause local adverse effects such as skin flare and itching at the injection site.

In the present invention, a biodegradable polymer which is conventionally used in the preparation of a microsphere can be used without limitation. For example, the biodegradable polymer includes, but is not limited to, polylactide, polyglycolide, poly(lactide-co-glycolide) and poly(lactide-co-glycolide)glucose.

In the present invention, the biodegradable polymer which has a weight average molecular weight of 60,000 or less may be used. For example, poly(lactide-co-glycolide)(50:50) having a molecular weight of about 13,000; poly(lactide-co-glycolide)(50:50) having a molecular weight of about 33,000; poly(lactide-co-glycolide)(50:50) having a molecular weight of about 52,000; poly(lactide-co-glycolide)(75:25) having a molecular weight of about 20,000; poly(lactide)(100:0) having a molecular weight of about 16,000 and the like may be used. Such a biodegradable polymer includes, for example, Resomer^{®} RG502H, RG503H, RG504H, RG752H, RG752S and R202H available from Boehringer Ingelheim.

In the present invention, the biodegradable polymer may have the intrinsic viscosity of 0.1 to 0.7 dL/g. In the present invention, if the intrinsic viscosity of the biodegradable polymer is less than 0.1 dL/g, the polymer degrades too quickly to control the continuous release of the physiologically active substance for the desired time. If the intrinsic viscosity of the biodegradable polymer exceeds 0.7 dL/g, the polymer degrades too slowly so that drug efficacy may not be shown due to the small amount of physiologically active substance released.

In the present invention, the biodegradable polymer may be comprised as the amount of 70.0 to 99.0% by weight, preferably 80.0 to 98.0% by weight, and more preferably 85.0 to 97.0% by weight based on the total weight of the microsphere. In the present invention, if the amount of the biodegradable polymer comprised in the microsphere is less than 70.0% by weight, there may be problems in suppressing the initial burst release or maintaining drug efficacy for the desired time due to the increase of the relative amount of physiologically active substance in the microsphere. If the amount of the biodegradable polymer comprised in the microsphere exceeds 99.0% by weight, the amount of microsphere to be injected into a patient is so large as to make it difficult or even impossible to inject.

In the above step (i) of the present invention, any of the non-aqueous solvents which are used in dissolving the biodegradable polymer may be used without limitation, so long as it dissolves the biodegradable polymer and is miscible with the co-solvent added in the above step (iii). For example, methylene chloride, chloroform, acetonitrile, dimethyl sulfoxide, dimethylformamide, ethyl acetate and the like may be used.

In the present invention, the co-solvent added in the above step (iii) is preferably miscible with the aqueous and non-aqueous solvent used in the above step (i), has a low solubility of physiologically active substance, and has a low boiling point to easily remove the remaining solvent in the process. In the present invention, examples of the co-solvent include, but are not limited to, methanol, ethanol, acetone, isopropanol, chloroform, diethyl ether, ethyl acetate, acetic acid, acetonitrile and mixtures thereof.

In the present invention, the co-solvent may be added in the amount of 1.0 to 20 parts by weight, preferably 5.0 to 15 parts by weight, more preferably 5.0 to 10 parts by weight based on 100 parts by weight of total weight of the solvent used in the above step (i), i.e., the aqueous solvent and the non-aqueous solvent. If the amount of the added co-solvent is less than 1.0% by weight, there may be no suppressive effect on the initial burst release. If the amount of the added co-solvent exceeds 20% by weight, it may be difficult to remove the remaining solvent.

In the present invention, it is believed that the addition of the co-solvent successfully suppresses the initial burst release by preventing the movement of physiologically active substance toward a surface at the time of evaporating a solvent by means of precipitating the physiologically active substance in a water phase and helping the hardness of biodegradable polymer to make emulsion relatively harder and reduce the porosity of the surface.

In the present invention, for the aqueous solvent used in the above step (iv), water for injection may be used, and optionally a low-viscosity polymer may be added thereto to suppress the dispersion of emulsion. Examples of low-viscosity polymer include, but are not limited to, polyvinylpyrrolidone, polyvinyl alcohol and the like.

### EFFECTS OF INVENTION

The present invention provides a method for preparing a sustained-release microsphere containing a physiologically active substance, specifically peptide or salt thereof, wherein the initial burst release is suppressed by adding a co-solvent which is removable in the preparation process, without requiring other additives and processes.

In addition, because the co-solvent used in the present invention is completely removed through the process of solvent removal, the present invention provides a sustained-release microsphere which is safe to the human body and has an excellent suppressive effect on the initial burst release.

### BRIEF DESCRIPTION OF FIGURES

Figure 1 is a photograph of the surface of the prepared microsphere (Before: before the use of co-solvent; After: after the use of co-solvent).
Figure 2 is a graph representing the results of *in vitro* long-term dissolution test (•: Example 5; ▲: Example 4; ×: Comparative Example).
Figure 3 is a graph representing the results of blood concentration of goserelin **(▲:** rat injected with microspheres).
Figure 4 is a graph representing the concentration change of testosterone after injection of goserelin ▲: hormone concentration of rat injected with microspheres; ○: hormone concentration of rat not injected with microspheres; ----: concentration of castration level).

### BEST MODE FOR CARRYING OUT INVENTION

Hereinafter, the present invention is explained in more detail with the following examples. However, it must be understood that the protection scope of the present invention is not limited to the examples.

### Examples 1 to 6

### Preparation of emulsion containing goserelin acetate

According to the content described in Table 1, 120 mg of goserelin acetate (Bachem, Switzerland) was added to 375 mg of water for injection and then dissolved by agitation to obtain a clear water phase. 1,250 mg of RG502H and 630 mg of RG503H (Boehringer Ingelheim) as biodegradable polymers, 5.0 mg of Span 80 (Merck) and 5,000 mg of methylene chloride (Merck) were dissolved by vigorous agitation, and then added to the water phase and vigorously agitated to form an emulsion.

**[Table 1]**

| Goserelin acetate (mg) | Water for injection (mg) | Biodegradable polymer | Amount (mg) | Methylene chloride (mg) | Surfactant (mg) |
|---|---|---|---|---|---|
| 120 | 375 | RG502H | 1,250 | 5,000 | 5.0 |
| | | RG503H | 630 | | |

### Preparation of microsphere according to the kind of co-solvent

According to the content described in Table 2, each co-solvent was added to the obtained emulsion above and then vigorously agitated. The obtained solution was slowly injected into 500 mℓ of 0.5% polyvinyl alcohol (Mn = 30,000 - 70,000; Sigma) solution vigorously agitated by an L4R mixer (Silverson, England) at 25°C. After 10 minutes, the temperature was raised to 40°C and the agitation rate was slowed down to evaporate the added organic solvent for 2 hours. The temperature was lowered to 25°C to allow cooling for 30 minutes, and vacuum filtration was carried out with a 5.0 µm filter (material: SVLP, Millipore). Then, the resultant was washed with distilled water several times and lyophilized for 72 hours to obtain a microsphere.

**[Table 2]**

| Example | Solvent | Amount (mg) |
|---|---|---|
| 1 | Ethanol | 450 |
| 2 | Acetone | 450 |
| 3 | Acetone | 225 |
| | Ethanol | 225 |
| 4 | Acetone | 360 |
| | Ethanol | 90 |
| 5 | Methanol | 450 |
| 6 | Isopropanol | 450 |

### Comparative Example 1

A microsphere was prepared according to the same method of Examples 1 to 6 except that the co-solvent was not added to the emulsion containing goserelin acetate prepared in the above Examples.

### Comparative Example 2

Commercially available goserelin acetate implant (Zoladex^{®}, AstraZeneca) was compared with the present microsphere under the same conditions.

### Experimental Example 1: Observation of Microsphere Morphology

To observe the surface of the microsphere, about 10 mg of the microsphere was fixed on an aluminum stub and coated with palladium under 0.1 torr of degree of vacuum and high voltage (10 kV) for 3 minutes. The palladium-coated microsphere was installed on a scanning electron microscope (SEM) (Hitachi S-4800 FE-SEM), and then the surface of the microsphere was observed by using an image-analysis program.

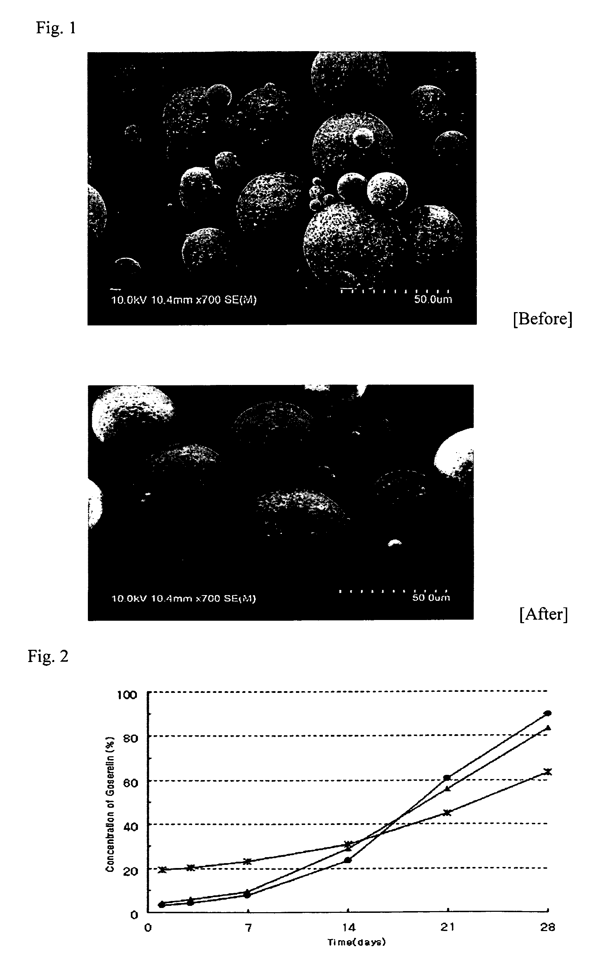

The results are represented in Figure 1. From the results, it can be known that the porosity of the surface is relatively decreased by using a co-solvent.

### Experimental Example 2: Measurement of Goserelin Loading Rate

About 100 mg of the microsphere was completely dissolved in 25 mℓ of dimethylformamide (Merck) and filtrated with a 0.45 µm syringe filter. The content of goserelin loaded into the microsphere was measured by HPLC under the following conditions.
Column: YMC C18 ODS 5 µm, 4.6×50 mm
Loading amount: 10 µℓ
Detection wavelength: 280 nm
Mobile phase: phosphate buffered saline (pH 3.0)

The results are represented in Table 3. From the results, it can be known that about 90% or more of goserelin based on the initial addition amount is sufficiently loaded into the microsphere.

**[Table 3]**

| | Goserelin Loading Rate (%) |
|---|---|
| Example 1 | 89.1 |
| Example 2 | 90.7 |
| Example 3 | 91.2 |
| Example 4 | 93.6 |
| Example 5 | 90.8 |
| Example 6 | 89.5 |

### Experimental Example 3: In vitro Long-term Dissolution Test of Microsphere

After placing about 50 mg of the microsphere into a 50 mℓ test tube, 50 mℓ of phosphate buffered saline (pH 7.4) was added thereto and incubated at 39°C. On days 1, 3, 7, 14, 21 and 28, supernatant was taken to measure the amount of goselerin released from the microsphere by HPLC. The measured results are represented in Table 4 and Figure 2.

From the measured results, it can be known that the initial dissolution rate of the microsphere in which co-solvent is added is lowered by half or more than that of the microsphere in which co-solvent is not added. It also can be known that the present microsphere suppresses the initial burst release in a level similar to that the implant formulation of Comparative Example 2 which has a low initial dissolution rate.

**[Table 4]**

| | Goserelin Dissolution Rate (%) | | | | | |
|---|---|---|---|---|---|---|
| | Day 1 | Day 3 | Day 7 | Day 14 | Day 21 | Day 28 |
| Example 1 | 8.7 | 12.5 | 15.2 | 25.3 | 42.5 | 70.3 |
| Example 2 | 7.1 | 8.9 | 10.3 | 20.1 | 40.9 | 75.0 |
| Example 3 | 4.2 | 5.8 | 9.3 | 28.8 | 55.9 | 83.3 |
| Example 4 | 3.3 | 4.2 | 7.8 | 23.4 | 60.7 | 89.6 |
| Example 5 | 5.2 | 5.9 | 7.1 | 19.3 | 44.0 | 78.8 |
| Example 6 | 7.8 | 10.6 | 12.8 | 26.7 | 50.3 | 73.6 |
| Comparative Example 1 | 19.2 | 20.3 | 23.1 | 30.8 | 45.0 | 63.5 |
| Comparative Example 2 | 3.4 | 4.5 | 7.3 | 29.6 | 80.4 | 99.4 |

### Experimental Example 4: In-vivo Test of Microsphere

The formulated goserelin was diluted in 1.5 mℓ of suspension solution, and then goserelin (100 µg/kg) was subcutaneously injected into 10 rats. At 6 hour, and days 1, 2, 4, 7, 14, 21 and 28 after injection, about 1.5 mℓ of blood sample was collected. The collected blood was centrifuged to obtain the plasma from the supernatant. The concentration of goserelin and sex hormone in the plasma was measured by using LC/MS/MS and a testosterone ELISA kit (IBL).

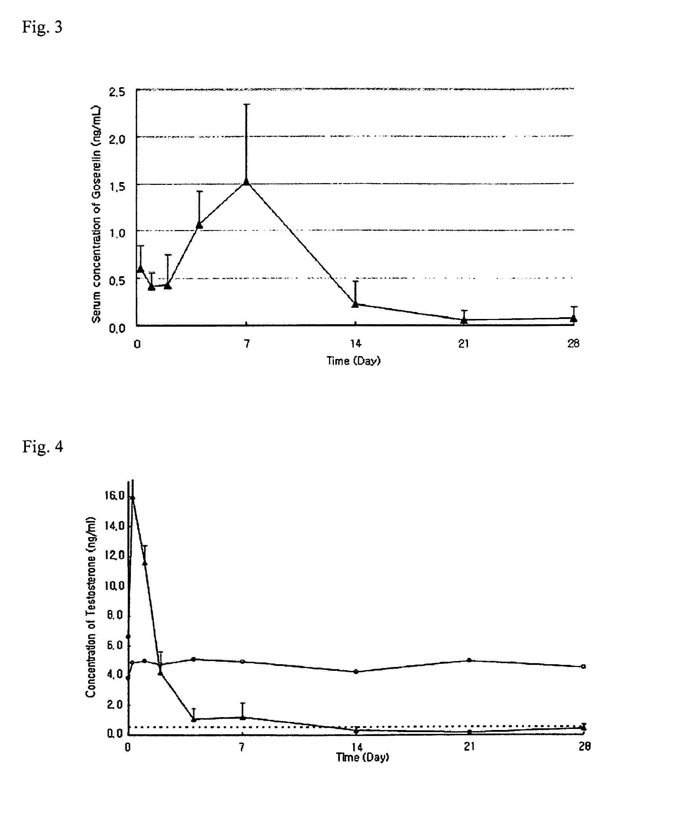

The measured results are represented in Figures 3 and 4. From the results, it can be known that the microsphere prepared according to the present invention continuously releases the drug in blood, and the concentration of hormone is maintained below the standard level.

## Claims

1. A method for preparing a sustained-release microsphere comprising the steps of:
i) dissolving a physiologically active substance and a biodegradable polymer in an aqueous solvent and a non-aqueous solvent, respectively, and then additionally dissolving a surfactant in the aqueous solvent, the non-aqueous solvent, or both the aqueous solvent and the non-aqueous solvent;
ii) forming an emulsion by combining the aqueous solution and the non-aqueous solution of step (i);
iii) adding a co-solvent to the emulsion formed in step (ii)
iv) forming a microsphere by adding the emulsion of step (iii) to an aqueous solvent; and
v) removing an organic solvent.

2. The method of claim 1, wherein the co-solvent is one or more selected from the group consisting of methanol, ethanol, acetone, isopropanol, chloroform, diethyl ether, ethyl acetate, acetic acid and acetonitrile.

3. The method of claim 1, wherein the co-solvent is added in the amount of 1.0 to 20 parts by weight based on 100 parts by weight of the aqueous solvent and the non-aqueous solvent of step (i).

4. The method of claim 1, wherein the physiologically active substance is one selected from goserelin, leuprolide, triptorelin, buserelin, octreotide and cetrorelix, or salt thereof.

5. The method of claim 4, wherein the content of the physiologically active substance is 1.0 to 30% by weight based on the total weight of the microsphere.

6. The method of claim 1, wherein the biodegradable polymer is selected from the group consisting of polylactide, polyglycolide, poly(lactide-co-glycolide) and poly(lactide-co-glycolide)glucose.

7. The method of claim 6, wherein the intrinsic viscosity of the biodegradable polymer is 0.1 to 0.7 dL/g.

8. The method of claim 6, wherein the content of the biodegradable polymer is 70.0 to 99.0% by weight based on the total weight of the microsphere.

9. The method of claim 1, wherein the surfactant is selected from the group consisting of polysorbate, Span, poloxamer, polyethylene glycol and tocopherol.

10. The method of claim 1, wherein the surfactant is added in the amount of 0.01 to 5.0 parts by weight based on 100 parts by weight of the aqueous solvent and the non-aqueous solvent of step (i).

11. A sustained-release microsphere which is prepared by the method according to any one of claims 1 to 10.
